# EUROPEAN PATENT APPLICATION

(11) **EP 4 393 467 A1**
(43) Date of publication of application: **03.07.2024**
(21) Application number: 22860538.2
(22) Date of filing: 24.08.2022
(51) Int. Cl.: A61J 1/20, A01N 1/02

(54) **DEVICE AND METHOD FOR FULLY ENCLOSED AND FULLY AUTOMATIC DISPENSING OF CELLS**

(30) Priority: 27.08.2021 CN 202110998251
(71) Applicant: AbelZeta Inc., Rockville, MD 20850 (US)
(72) Inventor: WANG, Fei, Shanghai 201210 (CN); XIE, Li, Shanghai 201210 (CN); ZHANG, Li, Shanghai 201210 (CN); REN, Jiaqiang, Shanghai 201210 (CN); ZHAO, Dijun, Shanghai 201210 (CN); ZHANG, Luyi, Shanghai 201210 (CN); ZHOU, Yiwen, Shanghai 201210 (CN)
(74) Representative: König Szynka Tilmann von Renesse Patentanwälte Partnerschaft mbB Düsseldorf
(86) International application number: PCT/CN2022/114503
(87) International publication number: WO 2023/025192

(57) **Abstract**

Disclosed are a device and method for fully enclosed and fully automatic dispensing of cells. The device includes a sample feeding module, a liquid replenishment module, and a liquid distribution module. By means of the device, cell dispensing can be performed under enclosed conditions, such that the risk of infection due to contact with an external environment is reduced. In addition, the survival rate of dispensed cells is ensured, and the degree of precision of cells obtained after dispensing is high, thereby improving the quality of the dispensed cells.

## Description

### FIELD OF TECHNOLOGY

The present invention relates to the technical field of biology, and specifically relates to a device and method for fully enclosed and fully automatic dispensing of cells.

### BACKGROUND

Cellular immunotherapy is a cell therapy for enhancing the targetability, lethality, and persistence of immune cells by collecting the immune cells of patients and performing gene modification and in vitro amplification culture. In recent years, the therapy has great clinical performance in tumor immunotherapy, which brings hope for clinical cure of tumors. However, the prepared immune cells need to be washed, concentrated, and dispensed into a plurality of freezing bags, and any intermediate link such as a process flow of dispensing cells, equipment and facilities, selection of reagents, and the like will affect the quality of dispensed cells, thereby affecting the clinical effect. Therefore, a process for fully enclosed and fully automatic dispensing of immune cells can ensure the product safety and the inter-batch stability, reduce the influence caused by humans and environments, and improve the quality of immune cell therapy products. Traditional cell dispensing can only be performed under artificial and non-enclosed conditions, thereby increasing the risk of infection due to contact with an external environment and greatly affecting the product safety.

Therefore, a method for fully enclosed and automatic dispensing of immune cells needs to be developed in the field, which can be used for rapidly obtaining high-quality immune cells for clinical use.

### SUMMARY

The purpose of the present invention is to provide a method for fully enclosed and automatic dispensing of immune cells, which can be used for rapidly obtaining high-quality immune cells for clinical use.

In a first aspect, the present invention provides a device for fully enclosed and fully automatic dispensing of cells. The device comprises a sample feeding module, a liquid replenishment module, and a liquid distribution module;

the sample feeding module comprises a cell sample container (such as a cell sample bag);
the liquid replenishment module comprises a plurality of liquid replenishers, and the liquid replenisher comprises a plurality of liquid replenishment containers (such as liquid replenishment bags (8-1, 8-2));
the liquid distribution module comprises a liquid distributor, the liquid dispenser is provided with a sample feeding pipe, a liquid outlet pipe, an extraction pipe, a waste liquid pipe, and a liquid replenishment pipe that are connected, the sample feeding pipe, the liquid outlet pipe, the extraction pipe, the waste liquid pipe, and the liquid replenishment pipe are communicated, and the sample feeding pipe, the liquid outlet pipe, the extraction pipe, the waste liquid pipe, and the liquid replenishment pipe are provided with a valve, respectively;
the sample feeding pipe is connected with the cell sample container (such as the cell sample bag), the liquid outlet pipe is connected with a cell freezing container (such as a cell freezing bag (7)), the extraction pipe is connected with an extraction device (1), the waste liquid pipe is connected with a waste liquid container (such as a waste liquid bag (6)), the liquid replenishment pipe is sequentially connected with the plurality of liquid replenishment containers (such as the liquid replenishment bags) through a plurality of branch pipes, and each branch pipe is provided with a valve.

In another preferred embodiment, the number of the liquid replenishment containers (such as the liquid replenishment bags) is 1, 2, 3, 4, 5, 6, 7, or 8.

In another preferred embodiment, the number of the liquid replenishment containers (such as the liquid replenishment bags) is two, and the two different liquid replenishment containers (such as the liquid replenishment bags) contain a liquid replenishment component 1 and a liquid replenishment component 2, respectively. Preferably, the liquid replenishment component 1 comprises a compound electrolyte injection and a human serum albumin aqueous solution, and the liquid replenishment component 2 comprises a CS10 cryopreservation solution.

In another preferred embodiment, the cell sample container (such as the cell sample bag) contains a cell sample.

In another preferred embodiment, the compound electrolyte injection comprises 3-7 parts by weight of sodium chloride, 3-7 parts by weight of sodium gluconate, 2-6 parts by weight of sodium acetate, 0.1-0.8 part by weight of potassium chloride, 0.1-0.6 part by weight of magnesium chloride, and 950-1,050 parts by weight of water.

In another preferred embodiment, the concentration of human serum albumin in the human serum albumin aqueous solution is 15-25% (v/v), preferably 18-22% (v/v).

In another preferred embodiment, the volume ratio of the compound electrolyte injection to the human serum albumin aqueous solution is (80-120):1, preferably (90-110):1, and more preferably (95-105):1.

In another preferred embodiment, the CS10 cryopreservation solution comprises a DMSO aqueous solution.

In another preferred embodiment, the CS10 cryopreservation solution comprises an 8-12% (v/v) DMSO aqueous solution.

In another preferred embodiment, the extraction device is capable of sucking a liquid and pumping a liquid.

In a second aspect, the present invention provides a method for preparing a cell suspension by using the device as described in the first aspect of the present invention. The method comprises the following steps:
(1) enabling the cell sample container (such as the cell sample bag) to contain a cell sample, and enabling two different liquid replenishment containers (such as liquid replenishment bags) to contain a liquid replenishment component 1 and a liquid replenishment component 2, respectively, wherein
   the liquid replenishment component 1 comprises a compound electrolyte injection and a human serum albumin aqueous solution, and the liquid replenishment component 2 comprises a CS10 cryopreservation solution;
(2) sending the cell sample in the cell sample container (such as the cell sample bag) into the extraction device by the sample feeding pipe and the extraction pipe based on suction of the extraction device, and then sending the cell sample into the waste liquid container (such as the waste liquid bag) by the extraction pipe and the waste liquid pipe based on discharge of the extraction device, so as to perform pipe rinsing on the sample feeding pipe, the extraction pipe and the waste liquid pipe;
(3) sending the cell sample in the cell sample container (such as the cell sample bag) into the extraction device by the sample feeding pipe and the extraction pipe based on suction of the extraction device, and then sending the cell sample into the cell freezing container (such as the cell freezing bag) by the extraction pipe and the liquid outlet pipe based on discharge of the extraction device;
(4) sending the liquid replenishment component 1 in the liquid replenishment container (such as the liquid replenishment bag) into the extraction device by the liquid replenishment pipe and the extraction pipe based on suction of the extraction device, and then sending the liquid replenishment component 1 into the cell freezing container (such as the cell freezing bag) by the extraction pipe and the liquid outlet pipe based on discharge of the extraction device;

and sending the liquid replenishment component 2 in the other liquid replenishment container (such as the other liquid replenishment bag) into the extraction device by the liquid replenishment pipe and the extraction pipe based on suction of the extraction device, and then sending the liquid replenishment component 2 into the cell freezing container (such as the cell freezing bag) by the extraction pipe and the liquid outlet pipe based on discharge of the extraction device;
wherein in step (4), the liquid replenishment component 1 and the liquid replenishment component 2 are sequentially sent into the cell freezing container (such as the cell freezing bag), and the volume ratio of the liquid replenishment component 1 to the liquid replenishment component 2 is 1:1.

In another preferred embodiment, the method further comprises a step (5): counting cells in the cell freezing container (such as the cell freezing bag) in step (4), performing liquid replenishment according to the operations in step (4) when the cell density is large, and replenishing the cell sample according to the operations in step (2) when the cell density is small, so as to obtain a cell suspension with a desired cell density.

In another preferred embodiment, the method further comprises a dispensing step (6), and the step comprises: connecting the cell freezing container (such as the cell freezing bag) containing the cell suspension with the sample feeding pipe, and connecting the waste liquid pipe with a dispensing bag;
the cell suspension in the cell freezing container (such as the cell freezing bag) is sent into the extraction device by the sample feeding pipe and the extraction pipe based on suction of the extraction device, then the cell suspension is sent into the dispensing bag by the extraction pipe and the waste liquid pipe based on discharge of the extraction device, and dispensing of the cell suspension for multiple times is achieved by changing the dispensing bag.

In another preferred embodiment, the dispensing step (6) is carried out after the step (5).

In another preferred embodiment, the compound electrolyte injection comprises 3-7 parts by weight of sodium chloride, 3-7 parts by weight of sodium gluconate, 2-6 parts by weight of sodium acetate, 0.1-0.8 part by weight of potassium chloride, 0.1-0.6 part by weight of magnesium chloride, and 950-1,050 parts by weight of water.

It is to be understood that, within the scope of the present invention, the above technical features of the present invention and technical features specifically described below (such as examples) can be combined with each other to form new or preferred technical schemes. Due to limited space, details are not described herein.

### BRIEF DESCRIPTION OF THE DRAWINGS

The drawings are used only for illustrative description and shall not be construed as limitations of the utility model. In order to better describe the examples, some parts in the drawings may be omitted, enlarged, or reduced, which do not represent the actual size of a product. For persons skilled in the art, it is understandable that some well-known structures and descriptions in the drawings may be omitted. Same or similar labels correspond to same or similar parts. Terms used for describing location relationships in the drawings are used only for illustrative description and shall not be construed as limitations of the present patent.

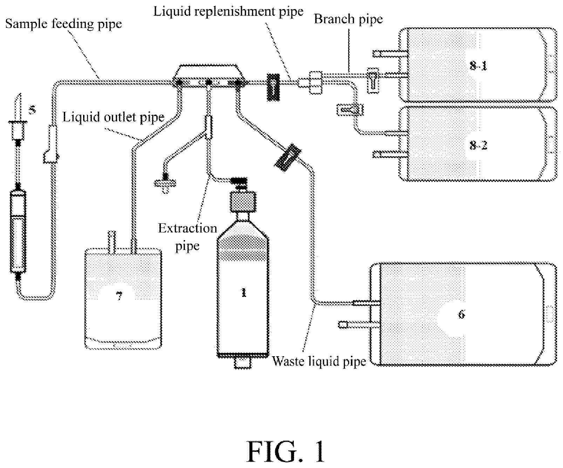

FIG. 1 is a structural schematic diagram of a device for fully enclosed and fully automatic dispensing of cells in an example, where 8-1 and 8-2 represent liquid replenishment bags, 5 represents a sample feeding pipe, 1 represents an extraction device, 6 represents a waste liquid bag, and 7 represents a cell freezing container (such as a cell freezing bag).

### DESCRIPTION OF THE EMBODIMENTS

Through extensive and in-depth research, the inventor has unexpectedly developed a device and method for fully enclosed and fully automatic dispensing of cells for the first time. By means of the method, cell dispensing can be performed under enclosed conditions, such that the risk of infection due to contact with an external environment is reduced. According to the device and method for fully enclosed and fully automatic dispensing of cells in the present invention, the survival rate of dispensed cells can be ensured, and the degree of precision of cells obtained after dispensing is high, thereby improving the quality of the dispensed cells. On this basis, the inventor has completed the present invention.

### Terms

Unless otherwise defined, all technical terms and scientific terms used herein have the same meanings as those generally understood by persons of ordinary skill in the field to which the present invention belongs.

As used herein, the terms "include", "comprise" and "contain" may be used interchangeably, which include not only open definitions, but also semi-closed and closed definitions. In other words, the terms include "consist of..." and "basically consist of...".

### Device

For convenient description, the device for fully enclosed and fully automatic dispensing of cells in the present invention is further described below with reference to FIG. 1. In the present invention, it is to be understood that the figure is not intended to limit the scope of the present invention.

Typically, the device for fully enclosed and fully automatic dispensing of cells in the present invention includes a sample feeding module, a liquid replenishment module, and a liquid distribution module;
the sample feeding module includes a cell sample container (such as a cell sample bag);
the liquid replenishment module includes a plurality of liquid replenishers, and the liquid replenisher includes a plurality of liquid replenishment containers (such as liquid replenishment bags (8-1, 8-2));
the liquid distribution module includes a liquid distributor, the liquid dispenser is provided with a sample feeding pipe, a liquid outlet pipe, an extraction pipe, a waste liquid pipe, and a liquid replenishment pipe that are connected, the sample feeding pipe, the liquid outlet pipe, the extraction pipe, the waste liquid pipe, and the liquid replenishment pipe are communicated, and the sample feeding pipe, the liquid outlet pipe, the extraction pipe, the waste liquid pipe, and the liquid replenishment pipe are provided with a valve, respectively;
the sample feeding pipe is connected with the cell sample container (such as the cell sample bag), the liquid outlet pipe is connected with a cell freezing container (such as a cell freezing bag (7)), the extraction pipe is connected with an extraction device (1), the waste liquid pipe is connected with a waste liquid container (such as a waste liquid bag (6)), the liquid replenishment pipe is sequentially connected with the plurality of liquid replenishment containers (such as the liquid replenishment bags) through a plurality of branch pipes, and each branch pipe is provided with a valve.

In one preferred embodiment of the present invention, the number of the liquid replenishment containers (such as the liquid replenishment bags) is 1, 2, 3, 4, 5, 6, 7, or 8.

In another preferred embodiment of the present invention, the number of the liquid replenishment containers (such as the liquid replenishment bags) is two, and the two different liquid replenishment containers (such as the liquid replenishment bags) contain a liquid replenishment component 1 and a liquid replenishment component 2, respectively. Preferably, the liquid replenishment component 1 includes a compound electrolyte injection and a human serum albumin aqueous solution, and the liquid replenishment component 2 includes a CS10 cryopreservation solution.

Preferably, the compound electrolyte injection includes 3-7 parts by weight of sodium chloride, 3-7 parts by weight of sodium gluconate, 2-6 parts by weight of sodium acetate, 0.1-0.8 part by weight of potassium chloride, 0.1-0.6 part by weight of magnesium chloride, and 950-1,050 parts by weight of water.

In another preferred embodiment, the concentration of human serum albumin in the human serum albumin aqueous solution is 15-25% (v/v), preferably 18-22% (v/v).

In another preferred embodiment, the volume ratio of the compound electrolyte injection to the human serum albumin aqueous solution is (80-120):1, preferably (90-110):1, and more preferably (95-105):1.

In another preferred embodiment, the CS10 cryopreservation solution includes a DMSO aqueous solution.

In another preferred embodiment, the CS10 cryopreservation solution includes an 8-12% (v/v) DMSO aqueous solution.

In another preferred embodiment of the present invention, the cell sample container (such as the cell sample bag) contains a cell sample.

### Method

The present invention further provides a method for dispensing cells by using the device for fully enclosed and fully automatic dispensing of cells in the present invention. The method includes the following steps:
(1) enabling the cell sample container (such as the cell sample bag) to contain a cell sample, and enabling two different liquid replenishment containers (such as liquid replenishment bags) to contain a liquid replenishment component 1 and a liquid replenishment component 2, respectively, wherein
   the liquid replenishment component 1 includes a compound electrolyte injection and a human serum albumin aqueous solution, and the liquid replenishment component 2 includes a CS10 cryopreservation solution;
(2) sending the cell sample in the cell sample container (such as the cell sample bag) into the extraction device by the sample feeding pipe and the extraction pipe based on suction of the extraction device, and then sending the cell sample into the waste liquid container (such as the waste liquid bag) by the extraction pipe and the waste liquid pipe based on discharge of the extraction device, so as to perform pipe rinsing on the sample feeding pipe, the extraction pipe and the waste liquid pipe;
(3) sending the cell sample in the cell sample container (such as the cell sample bag) into the extraction device by the sample feeding pipe and the extraction pipe based on suction of the extraction device, and then sending the cell sample into the cell freezing container (such as the cell freezing bag) by the extraction pipe and the liquid outlet pipe based on discharge of the extraction device;
(4) sending the liquid replenishment component 1 in the liquid replenishment container (such as the liquid replenishment bag) into the extraction device by the liquid replenishment pipe and the extraction pipe based on suction of the extraction device, and then sending the liquid replenishment component 1 into the cell freezing container (such as the cell freezing bag) by the extraction pipe and the liquid outlet pipe based on discharge of the extraction device;

and sending the liquid replenishment component 2 in the other liquid replenishment container (such as the other liquid replenishment bag) into the extraction device by the liquid replenishment pipe and the extraction pipe based on suction of the extraction device, and then sending the liquid replenishment component 2 into the cell freezing container (such as the cell freezing bag) by the extraction pipe and the liquid outlet pipe based on discharge of the extraction device;
wherein in step (4), the liquid replenishment component 1 and the liquid replenishment component 2 are sequentially sent into the cell freezing container (such as the cell freezing bag), and the volume ratio of the liquid replenishment component 1 to the liquid replenishment component 2 is 1:1.

In one preferred embodiment of the present invention, the compound electrolyte injection includes 3-7 parts by weight of sodium chloride, 3-7 parts by weight of sodium gluconate, 2-6 parts by weight of sodium acetate, 0.1-0.8 part by weight of potassium chloride, 0.1-0.6 part by weight of magnesium chloride, and 950-1,050 parts by weight of water.

In one preferred embodiment of the present invention, the method further includes a step (5): counting cells in the cell freezing container (such as the cell freezing bag) in step (4), performing liquid replenishment according to the operations in step (4) when the cell density is large, and replenishing the cell sample according to the operations in step (2) when the cell density is small, so as to obtain a cell suspension with a desired cell density.

In one preferred embodiment of the present invention, the method further includes a dispensing step (6), and the step includes: connecting the cell freezing container (such as the cell freezing bag) containing the cell suspension with the sample feeding pipe, and connecting the waste liquid pipe with a dispensing bag;

the cell suspension in the cell freezing container (such as the cell freezing bag) is sent into the extraction device by the sample feeding pipe and the extraction pipe based on suction of the extraction device, then the cell suspension is sent into the dispensing bag by the extraction pipe and the waste liquid pipe based on discharge of the extraction device, and dispensing of the cell suspension for multiple times is achieved by changing the dispensing bag.

In another preferred embodiment, the dispensing step (6) is carried out after the step (5).

### The present invention mainly has the following advantages:

According to the device and method for fully enclosed and fully automatic dispensing of cells in the present invention, cell dispensing can be performed under enclosed conditions, such that the risk of infection due to contact with an external environment is reduced. According to the device and method for fully enclosed and fully automatic dispensing of cells in the present invention, the survival rate of dispensed cells can be ensured, and the degree of precision of cells obtained after dispensing is high, thereby improving the quality of the dispensed cells.

By means of the method for preparing cells in the present invention, immune cells can be rapidly prepared, the cost of an enterprise is reduced, the production capacity is improved, and the method is suitable for industrial production. Meanwhile, the immune cells prepared by the method for preparing cells in the present invention have high quality, such that the clinical effect can be ensured.

The present invention is further illustrated below in combination with specific examples. It is understood that these examples are used only to describe the present invention and are not intended to limit the scope of the present invention. Experimental methods without specific conditions in the following examples are usually used in accordance with conventional conditions, or in accordance with conditions recommended by manufacturers. Unless otherwise specified, percentages and parts are calculated by weight.

### Example 1

### 1. Experimental scheme

a) Demands of cryopreserved cell quantities: The quantity of positive T cells in a final product bag was 150×10⁶ cells, and the cryopreservation volume was 40 ml; the quantity of positive T cells in a test cell bag was 37.5×10⁶ cells, and the cryopreservation volume was 10 ml; the cell positive rate was 20.71%; and the theoretical cryopreservation density was 19.918×10⁶ cells/ml.
b) Preparation of functional reagents:
   i. Component 1: An HSA (human serum albumin) aqueous solution with a concentration of 20% (v/v) was added into a compound electrolyte injection (each 1,000 ml of water containing 5.26 g of sodium chloride, 5.02 g of sodium gluconate, 3.68 g of sodium acetate, 0.37 g of potassium chloride, and 0.30 g of magnesium chloride), and then filled into a component 1 bag; where the volume ratio of the HSA aqueous solution with a concentration of 20% (v/v) to the compound electrolyte injection was 1:100.
   ii. Component 2: A CS10 cryopreservation solution was filled into a component 2 bag, where the CS10 cryopreservation solution was a DMSO aqueous solution, and the volume fraction of DMSO was 10%.
c) Pipe preparation before dispensing:
   i. Kit CT-49.1 (device for fully enclosed and fully automatic dispensing of cells) was opened in a safety cabinet, a clamp was closed, and a sterile tube connect was used for connecting a harvested cell sample bag at position 5, a 250 ml cell freezing bag at position 7, the component 1 bag at position 8-1, the component 2 bag at position 8-2 and a waste liquid bag at position 6, respectively. A pipe position schematic diagram of the Kit CT-49.1 is shown in FIG. 1.
   ii. Pipes are installed on Sepax according to FIG. 1.
d) Concentration of a cell suspension by a Culture Wash procedure
   i. Cell counting: After concentration of a cell suspension, a 5 ml syringe was connected to the 250 ml freezing bag containing the cell suspension, the freezing bag was shaken uniformly, and about 0.2 ml of the cell suspension was sucked for cell counting.
e) Preparation of a final product by a Dilution (dispensing) procedure: A final cryopreservation solution was added with the component 1 and the component 2 at a ratio of 50%:50%.
   i. Component volume calculation: According to the cell counting result in the above step, the cell suspension volume, the final product filling density and the final product filling cell quantity, the added volume of the component 1 and the component 2 was calculated, respectively. The added volume of the component 1=the volume of the cell suspension in the freezing bag + the volume of the component 1 that further needs to be added.
   ii. Pipe rinsing: The rinsing volume, 10 ml, of the component 1 was imputed, a pipe clamp was opened, and a procedure was started.
   iii. Dispensing of the component 1: The dispensing volume, namely the volume of the component 1 that further needs to be added, was imputed, "✔" was clicked, a pipe clamp was opened, and a procedure was started.
   iv. Pipe rinsing: The rinsing volume, 10 ml, of the component 2 was imputed, a pipe clamp was opened, and a procedure was started.
   v. Dispensing of the component 2: The dispensing volume, namely the total volume of the component 2 that needs to be added, was imputed, "✔" was clicked, a pipe clamp was opened, and a procedure was started.
f) Dispensing of the final product:
   i. Pipe rinsing: The rinsing volume, 10 ml, of the final product was imputed, a pipe clamp was opened, and a procedure was started.
   ii. Dispensing of a test cell bag: Before dispensing, the dispensing volume was calculated first according to the filling cell quantity and cryopreservation density of the test cell bag, then a 50 ml cell freezing bag was connected to position 6, the calculated dispensing volume was imputed, "✔" was clicked, a pipe clamp was opened, and a procedure was started. The step was repeated twice to obtain three test cell bags.
   iii. Dispensing of the final product: Before dispensing, the dispensing volume was calculated first according to the filling cell quantity and cryopreservation density of a final product bag, then a 250 ml cell freezing bag was connected to position 6, the calculated dispensing volume was imputed, "✔" was clicked, a pipe clamp was opened, and a procedure was started. Finally, one final product bag was obtained.
   iv. Air in the three test cell bags and the final product bag was exhausted, and the bags were sealed.
   v. The sealed bags were transferred into a controlled-rate freezer, frozen by the controlled-rate freezer, and then transferred into a liquid nitrogen refrigerator for storage after freezing.

### 2. Experimental results

a) Counting of the concentrated cell suspension
The density and viability of concentrated cells in the cell sample bag were measured by a cell counter, as shown in Table 1. The cell quantity after concentration was expressed as 181.5×10⁶ cells/ml ×(12.2 mL-0.2 ml)=2178×10⁶ cells.

**Table 1. Counting of a concentrated cell suspension**

| Cell counting | Cell density (×10⁶ cells/ml) | Cell viability (%) |
|---|---|---|
| 1 | 185 | 96.0 |
| 2 | 178 | 95.9 |
| Average | 181.5 | 96.0 |

b) Preparation of the final product
The calculated component volume is shown in Table 2, and the total volume is expressed as 54.7 ml+54.7 ml=109.4 ml.

**Table 2. Component volume**

| Component name | Dispensing volume (ml) |
|---|---|
| Component 1 | 54.7 |
| Component 2 | 54.7 |

c) Dispensing of the final product

The density and viability of cells dispensed in the three test cell bags and the final product bag were measured by a cell counter, as shown in Table 4, Table 5, Table 6 and Table 7, respectively. The positive cell quantity of the test cell bag 1 was expressed as 19.25×10⁶ cells/ml×10 ml×20.71%=39.87×10⁶ cells. The positive cell quantity of the test cell bag 2 was expressed as 18.8×10⁶ cells/ml×10 ml×20.71%=38.93×10⁶ cells. The positive cell quantity of the test cell bag 3 was expressed as 16.5×10⁶ cells/ml×10 ml×20.71%=34.17×10⁶ cells. The positive cell quantity of the final product bag was expressed as 20.033×10⁶ cells/ml×40 ml×20.71%=165.95×10⁶ cells. Summary results of cell dispensing are shown in Table 8. Compared with the theoretical freezing density, the cell density of the three test cell bags and the final product bag has no great difference, and the maximum difference is 1517.16%. Compared with the theoretical positive cell quantity, the maximum difference is 10.63%, which is within an acceptable range, and the various bags almost have no difference in cell viability. Therefore, the volume and total cell quantity of the final product prepared by Sepax using the process are accurate, and the cells are barely lost, indicating that it is possible to use Sepax to perform final product dispensing on the collected cells, and fully enclosed automation of a process flow of dispensing immune cells is realized.

**Table 4. Cell counting of a test cell bag 1**

| Cell counting | Cell density (×10⁶ cells/ml) | Cell viability (%) |
|---|---|---|
| 1 | 19.3 | 93.3 |
| 2 | 19.2 | 93.0 |
| Average | 19.25 | 93.2 |

**Table 5. Cell counting of a test cell bag 2**

| Cell counting | Cell density (×10⁶ cells/ml) | Cell viability (%) |
|---|---|---|
| 1 | 18.9 | 92.7 |
| 2 | 18.7 | 89.7 |
| Average | 18.8 | 91.2 |

**Table 6. Cell counting of a test cell bag 3**

| Cell counting | Cell density (×10⁶ cells/ml) | Cell viability (%) |
|---|---|---|
| 1 | 16.5 | 93.7 |
| 2 | 16.5 | 92.6 |
| Average | 16.5 | 93.2 |

**Table 7. Counting of a final product**

| Cell counting | Cell density (×10⁶ cells/ml) | Cell viability (%) |
|---|---|---|
| 1 | 22.6 | 92.9 |
| 2 | 18.7 | 91.6 |
| 3 | 18.8 | 91.9 |
| Average | 20.033 | 92.1 |

**Table 8. Summary results of cell dispensing**

| Bag number | Cell density (×10⁶ cells/ml) | Cell viability (%) | Output volume (ml) | Actual output of positive cell quantity (×10⁶ cells/ml) | Fluctuation of cell density (%) | Fluctuation of positive cell quantity (%) |
|---|---|---|---|---|---|---|
| Test bag 1 | 19.25 | 93.2 | 10 | 39.87 | 3.35 | 6.32 |
| Test bag 2 | 18.8 | 91.2 | 10 | 38.93 | 5.61 | 3.81 |
| Test bag 3 | 16.5 | 93.2 | 10 | 34.17 | 17.16 | 8.88 |
| Final product bag | 20.033 | 92.1 | 80 | 165.95 | 0.58 | 10.63 |

All documents mentioned in the present invention are cited as references in the present application as if each document is cited separately as a reference. In addition, it is to be understood that after reading the above contents of the present invention, persons skilled in the art may make various changes or modifications to the present invention, and all the equivalent forms also fall within the scope limited by the claims of the present application.

## Claims

1. A device for fully enclosed and fully automatic dispensing of cells, **characterized by** comprising a sample feeding module, a liquid replenishment module, and a liquid distribution module;
wherein the sample feeding module comprises a cell sample container;
the liquid replenishment module comprises a plurality of liquid replenishers, and the liquid replenisher comprises a plurality of liquid replenishment containers;
the liquid distribution module comprises a liquid distributor, the liquid dispenser is provided with a sample feeding pipe, a liquid outlet pipe, an extraction pipe, a waste liquid pipe, and a liquid replenishment pipe that are connected, the sample feeding pipe, the liquid outlet pipe, the extraction pipe, the waste liquid pipe, and the liquid replenishment pipe are communicated, and the sample feeding pipe, the liquid outlet pipe, the extraction pipe, the waste liquid pipe, and the liquid replenishment pipe are provided with a valve, respectively;
the sample feeding pipe is connected with the cell sample container, the liquid outlet pipe is connected with a cell freezing container, the extraction pipe is connected with an extraction device, the waste liquid pipe is connected with a waste liquid container, the liquid replenishment pipe is sequentially connected with the plurality of liquid replenishment containers through a plurality of branch pipes, and each branch pipe is provided with a valve.

2. The device according to claim 1, wherein the number of the liquid replenishment containers is two, and the two different liquid replenishment containers contain a liquid replenishment component 1 and a liquid replenishment component 2, respectively, wherein the liquid replenishment component 1 comprises a compound electrolyte injection and a human serum albumin aqueous solution, and the liquid replenishment component 2 comprises a CS10 cryopreservation solution.

3. The device according to claim 2, wherein the compound electrolyte injection comprises 3-7 parts by weight of sodium chloride, 3-7 parts by weight of sodium gluconate, 2-6 parts by weight of sodium acetate, 0.1-0.8 part by weight of potassium chloride, 0.1-0.6 part by weight of magnesium chloride, and 950-1,050 parts by weight of water.

4. The device according to claim 2, wherein the concentration of human serum albumin in the human serum albumin aqueous solution is 15-25% (v/v).

5. The device according to claim 1, wherein the extraction device is capable of sucking a liquid and pumping a liquid.

6. The device according to claim 2, wherein the volume ratio of the compound electrolyte injection to the human serum albumin aqueous solution is (80-120):1, preferably (90-110):1, and more preferably (95-105):1.

7. The device according to claim 2, wherein the CS10 cryopreservation solution comprises an 8-12% (v/v) DMSO aqueous solution.

8. A method for preparing a cell suspension by using the device according to claim 1, **characterized by** comprising the following steps:
(1) enabling the cell sample container to contain a cell sample, and enabling two different liquid replenishment containers to contain a liquid replenishment component 1 and a liquid replenishment component 2, respectively, wherein
the liquid replenishment component 1 comprises a compound electrolyte injection and a human serum albumin aqueous solution, and the liquid replenishment component 2 comprises a CS10 cryopreservation solution;
(2) sending the cell sample in the cell sample container into the extraction device by the sample feeding pipe and the extraction pipe based on suction of the extraction device, and then sending the cell sample into the waste liquid container by the extraction pipe and the waste liquid pipe based on discharge of the extraction device, so as to perform pipe rinsing on the sample feeding pipe, the extraction pipe and the waste liquid pipe;
(3) sending the cell sample in the cell sample container into the extraction device by the sample feeding pipe and the extraction pipe based on suction of the extraction device, and then sending the cell sample into the cell freezing container by the extraction pipe and the liquid outlet pipe based on discharge of the extraction device;
(4) sending the liquid replenishment component 1 in the liquid replenishment container into the extraction device by the liquid replenishment pipe and the extraction pipe based on suction of the extraction device, and then sending the liquid replenishment component 1 into the cell freezing container by the extraction pipe and the liquid outlet pipe based on discharge of the extraction device;
and sending the liquid replenishment component 2 in the other liquid replenishment container into the extraction device by the liquid replenishment pipe and the extraction pipe based on suction of the extraction device, and then sending the liquid replenishment component 2 into the cell freezing container by the extraction pipe and the liquid outlet pipe based on discharge of the extraction device;
wherein in step (4), the liquid replenishment component 1 and the liquid replenishment component 2 are sequentially sent into the cell freezing container, and the volume ratio of the liquid replenishment component 1 to the liquid replenishment component 2 is 1:1.

9. The method according to claim 8, further comprising a step (5): counting cells in the cell freezing container in step (4), performing liquid replenishment according to the operations in step (4) when the cell density is large, and replenishing the cell sample according to the operations in step (2) when the cell density is small, so as to obtain a cell suspension with a desired cell density.

10. The method according to claim 8, further comprising a dispensing step (6), wherein the step comprises: connecting the cell freezing container containing the cell suspension with the sample feeding pipe, and connecting the waste liquid pipe with a dispensing bag;
the cell suspension in the cell freezing container is sent into the extraction device by the sample feeding pipe and the extraction pipe based on suction of the extraction device, then the cell suspension is sent into the dispensing bag by the extraction pipe and the waste liquid pipe based on discharge of the extraction device, and dispensing of the cell suspension for multiple times is achieved by changing the dispensing bag.

11. The method according to claim 8, wherein the compound electrolyte injection comprises 3-7 parts by weight of sodium chloride, 3-7 parts by weight of sodium gluconate, 2-6 parts by weight of sodium acetate, 0.1-0.8 part by weight of potassium chloride, 0.1-0.6 part by weight of magnesium chloride, and 950-1,050 parts by weight of water.
